# EUROPEAN PATENT SPECIFICATION

(11) **EP 3 149 158 B1**
(45) Date of publication and mention of the grant of the patent: **11.10.2017**
(21) Application number: 15798229.9
(22) Date of filing: 04.11.2015
(51) Int. Cl.: C12N 7/00, A61K 39/15

(54) **LIVE ATTENUATED AFRICAN HORSESICKNESS VIRUS**
LEBENDES ABGESCHWÄCHTES AFRIKANISCHES PFERDEPESTVIRUS
VIRUS DE LA PESTE ÉQUINE VIVANT ATTÉNUÉ

(30) Priority: 04.11.2014 ZA 201408067
(43) Date of publication of application: 05.04.2017
(73) Proprietor: Deltamune (PTY) Limited, 0157 Pretoria (ZA)
(72) Inventor: POTGIETER, Abraham, Christiaan, 0157 Pretoria (ZA); WRIGHT, Isabella, Maria, 0186 Pretoria (ZA); ERASMUS, Balthasar, Johannes, 6665 Heidelberg, Western Cape (ZA)
(74) Representative: Von Rohr Patentanwälte Partnerschaft mbB
(86) International application number: PCT/IB2015/058522
(87) International publication number: WO 2016/071850

(56) References cited:
- WO-A2-2010/048394
- Y. KANAME ET AL: "Recovery of African horse sickness virus from synthetic RNA", JOURNAL OF GENERAL VIROLOGY., vol. 94, no. Pt_10, 1 October 2013 (2013-10-01), pages 2259-2265, XP055244679, GB ISSN: 0022-1317, DOI: 10.1099/vir.0.055905-0 cited in the application
- MAXIME RATINIER ET AL: "Identification and Characterization of a Novel Non-Structural Protein of Bluetongue Virus", PLOS PATHOGENS, vol. 7, no. 12, 29 December 2011 (2011-12-29), page e1002477, XP055134533, DOI: 10.1371/journal.ppat.1002477 cited in the application
- LIZAHN ZWART ET AL: "Characterising Non-Structural Protein NS4 of African Horse Sickness Virus", PLOS ONE, vol. 10, no. 4, 27 April 2015 (2015-04-27) , page e0124281, XP055243647, DOI: 10.1371/journal.pone.0124281

## Description

The present invention relates to a live attenuated African horsesickness virus, (family *Reoviridae*, genus *Orbivirus*), a method of producing and using such a virus, a vaccine and an immunisation kit including such a virus.

African horsesickness ("AHS") is a serious disease of *Equidae* which is enzootic in sub-Saharan Africa and caused by African horsesickness virus ("AHSV").

There are nine distinct serotypes of AHSV, which can be distinguished in virus or serum neutralisation tests (VNT or SNT) (Mclntosh, 1958). The identity of each serotype is controlled primarily by the amino acid sequence of the major outer capsid protein namely VP2, which contains the majority of neutralising epitopes and is the principal serotype-specific antigen of AHSV (Burrage, 1993). Animals that survive infection by a single AHSV serotype are subsequently protected against the homologous type, although such animals are still susceptible to most other serotypes.

The AHSV core consists of two major proteins that form distinct capsid layers: VP7 forms the core surface layer; while VP3 forms the innermost 'sub-core' shell. The sub-core also contains three minor proteins, VP1, VP4 and VP6 that form core associated transcriptase complexes, and surround the 10 segments of the viral genome (numbered segment 1 to segment 10 [S1 to S10] in order of decreasing molecular weight) (Roy *et al.,* 1994).

AHS causes a high mortality rate (up to 95%) among naive horses, and there is no cure or specific treatment for the disease. AHS is notifiable to the World Organisation for Animal Health (OIE). AHS significantly impedes the trade and movement of horses to and from South Africa, especially when AHS-free countries are involved. In South Africa, outbreaks of AHS occur annually and all nine serotypes of the virus circulate in the country. The virus has emerged several times in non-endemic regions, which has drawn focus to the epizootiology, molecular biology and control of the virus (Mellor and Boorman, 1995).

AHSV resembles the orbivirus type species, bluetongue virus (BTV), in many respects. The genome of the virus consists of ten double stranded ribonucleic acid ('dsRNA') segments which encode seven known viral proteins (VP 1 - 7) which form part of the structure of the virus, as well as four known non-structural proteins which are expressed during the infection cycles in the cell (NS1, NS2, NS3 and NS3a), but do not form part of the virus structure.

Recent bio-informatics analysis of the genome of orbiviruses showed that the segment that encodes VP6 also encodes a fifth non-structural protein, which has been designated NS4. NS4 is expressed in bluetongue virus (BTV) and Great Island virus (GIV) infected mammalian cells. NS4 in cells infected with these viruses localises to the nucleolus, cell membrane and cytoplasm at various stages of infection. GIV NS4 binds double-stranded DNA and double stranded RNA (dsDNA and dsRNA), whereas BTV NS4 binds dsDNA only. BTV NS4 also appears to be involved in modulating host innate immunity during infection with some serotypes (Belhouchet *et al*., 2011; Firth 2008; Ratinier *et al*., 2011). The exact function of NS4 in the replication cycles of these viruses has not yet been published, although its higher level of conservation compared to the overlapping VP6 gene is suggestive of functional significance. Previous analysis of genome segment 9 sequences of AHSV-3 and AHSV-6 showed a 97% nucleotide identity and a 95% amino acid identity of the cognate VP6 protein (Turnbull *et al.,* 1996).

The complete AHSV NS4 protein has a size of 16 or 18 kDa, depending on the particular strain and contains three potential overlapping nuclear localisation signals (NLSs) which predict a dual cytoplasmic and nuclear localisation, and has putative nucleic acid binding or modifying domains (Belhouchet *et al.* 2011; Firth 2008). Some details of the expression of NS4 in AHSV infected cell culture and in host animals (horses) have been published by Zwart *et al.,* 2015. AHSV NS4 localises in the nucleus of infected cells, but not in the nucleolus as in the case of BTV NS4. Recombinant AHSV NS4 binds dsDNA non-specifically, but it does not bind to dsRNA. Rabbit anti-AHSV NS4 antibody immune-staining of organs from horses that died of AHS, shows that NS4 is abundantly expressed in stellate cells and more specifically stellate cells in the spleen. These stellate cells may be dendritic cells and this suggests that AHSV NS4 functions specifically in immune cells.

The most important measure for the control of AHS is vaccination. A known AHSV vaccine is a live-attenuated vaccine (modified-live virus, MLV), administered in two vaccinations that collectively protect against all 9 serotypes (cross protection is expected to occur for serotypes 5 and 9 by serotypes 8 and 6 respectively).

Furthermore, the WO 2010/048394 A2 describes vectors that contain and express in vivo the genes encoding VP2 and VP5 of African Horse Sickness Virus or an epitope thereof that elicits an immune response in a horse against African Horse Sickness Virus, compositions comprising said vectors, methods of vaccination against African Horse Sickness Virus and kits for use with such methods and compositions.

Although AHS has been ameliorated by this vaccination strategy, outbreaks still occur and the known vaccine suffers from various disadvantages.

One disadvantage of the known vaccine is the potential of the vaccine to revert to a virulent strain through re-assortment with segments from virulent strains during an outbreak. A further disadvantage is that live attenuated vaccine viruses could revert back to pathogenicity through re-assortment with other live attenuated strains.

Yet another disadvantage of the current vaccine is that the vaccine can be spread by vaccinated immune-compromised hosts. There may be a requirement for repeated vaccinations in some animals due to low immunogenicity of the current vaccine. A study on field collected vectors showed that *C*. *imicola* and *C*. *bolitinos* are susceptible to experimental infection with AHSV vaccine strains, although the virus replication rate was reduced within the insect hosts and may not be sufficient for transmission to vertebrate hosts in the field. Therefore, if transmission is possible, vaccination with certain live attenuated strains may potentially result in AHS outbreaks (Paweska *et al.,* 2003). For example, a virus strain (serotype 9), that was probably derived from the live attenuated vaccine serotype 9 strain, was isolated from a horse in Gambia, supporting the notion that vaccine strains may spread amongst susceptible hosts or revert to virulence by means of re-assortment (Oura *et al*., 2011). A study in South Africa showed that 16% of ponies immunised with a live-attenuated polyvalent vaccine became naturally infected with AHSV within two years. Half of these animals were sub-clinically infected (Weyer *et al.,* 2013).

There is thus a need for stable and safe reverse genetic engineered AHSV for use as vaccines.

Considering the susceptibility of animals to AHSV, a method of immunising animals against AHS is essential.

The present invention aims to reduce or eliminate some or all of the known disadvantages associated with the known attenuated AHSV strains, and in particular with the generation of a live attenuated recombinant AHSV.

It is accordingly an object of the present invention to provide a live attenuated recombinant AHSV, a method of producing such a virus, a vaccine and a kit comprising the virus with which the aforesaid disadvantages could be overcome or at least minimised.

According to a first aspect of the invention there is provided a live attenuated recombinant African horsesickness virus (AHSV) of which the genome has been altered such that the expression of the non-structural protein 4 (NS4) as a wild-type or complete protein is eliminated.

Further according to the invention, the genome of the recombinant virus has been altered such that the expression of NS4 as a wild-type or complete protein has been eliminated by substitution of at least one start (initiation) codon in the open reading frame encoding for NS4, without affecting the functioning of virus protein 6 (VP6) of the virus. Alternatively, said start codon is deleted or otherwise changed so as to effect said elimination, without affecting the functioning of virus protein 6 (VP6) of the virus.

Preferably from one to ten start codons of the open reading frame encoding for NS4 have been substituted or otherwise changed without affecting the functioning of VP6 of the virus.

The AHSV may be selected from the group consisting of AHSV serotype 1 to AHSV serotype 9 and attenuated AHSV strains.

Further according to the invention, the recombinant virus of a particular serotype or strain of AHSV includes a genome segment 2 encoding the serotype determining viral protein (VP2) and/or genome segment 6 encoding VP5 of another AHSV serotype or strain.

According to a second aspect of the invention there is provided a method for producing a non-pathogenic live attenuated recombinant AHSV, the method including the steps of:
- selecting a particular strain of AHSV;
- determining the genome sequence of the selected AHSV strain;
- altering the genome sequence of the AHSV strain such that the expression of the non-structural protein 4 (NS4) as a wild-type or complete protein is eliminated;
- synthesising transcription plasmids containing the altered genome sequence;
- synthesising expression plasmids containing codon-optimised open reading frames of the proteins to provide a replication complex;
- synthesising single stranded RNA (ssRNA) from the said transcription plasmids;
- transfecting a permissive cell line with the said expression plasmids;
- subsequently transfecting the said transfected permissive cell line with ssRNA; and
- allowing the incorporation of the ssRNA into the replication complex in the transfected permissive cell line to produce replicating live attenuated recombinant AHSV.

The method may include the further step of re-sequencing the genome of the produced recombinant AHSV to confirm that the alteration to the genome has been effected.

The method may include the further step of replacing the synthetic ssRNA of the genome segments 2 and 6 encoding the serotype determining viral proteins (VP2 and VP5) with that of another AHSV serotype.

According to a third aspect of the invention there is provided a vaccine comprising the recombinant AHSV according to the first aspect of the invention.

The vaccine may optionally further include a pharmaceutically acceptable carrier, adjuvant or vehicle.

The pharmaceutically acceptable carrier may be selected from the group consisting of water based adjuvant and liquid nano-particles containing an immune-stimulating compound and a combination thereof. Typically the pharmaceutical carrier is selected from the commercially available carriers such as SEPPIC MONTANIDE™ Pet Gel, SEPPIC MONTANIDE™ IMS 3012 and/or equivalents or combinations thereof.

The vaccine may be administered in any one or more methods selected from the group consisting of subcutaneous, intramuscular, or intradermal.

The vaccine may be in the form of an injectable preparation, including an injectable aqueous suspension.

According to another aspect of the invention there is provided use of a vaccine according to the third aspect of the invention in a method of vaccinating animals against AHS.

According to another aspect of the present invention there is provided a method for vaccinating an animal against AHS including the step of delivering an effective amount of the vaccine according to the third aspect of the invention to the animal.

According to another aspect of the present invention there is provided a multivalent vaccine, comprising a recombinant vaccine virus of one serotype and at least one other serotype, both according to the first aspect of the invention.

According to yet another aspect of the invention there is provided an immunisation kit comprising two containers each including at least four different serotypes of recombinant AHSV according to the first aspect of the invention, the arrangement being such that the kit is suitable for use in a method of immunising an animal against all nine serotypes of AHSV.

The invention will now be described further, by way of example only, with reference to accompanying figures and photographs wherein:
- Figure 1: is a representation of a T7 AHSV plasmid clone having a T7 promoter upstream of each of the full length AHSV genome segments with a unique restriction enzyme site such as *BsmBI*, downstream of the genomic segment, illustrating a step in a method according to a preferred embodiment of the invention of producing a non-pathogenic recombinant vaccine virus of AHSV;
- Figure 2: is a representation of a step in the method of figure 1, illustrating the generation of an AHSV ssRNA from the T7 transcription plasmid digested with a restriction enzyme, by incubating T7 digested plasmid with a T7 polymerase, buffers and nucleotides;
- Figure 3: is a representation of an expression plasmid clone having a Cytomegalovirus (CMV) DNA dependent RNA polymerase II promoter upstream of the reading frame of each of the AHSV genes that encode the proteins that constitute the replication complex of AHSV with the poly-T sequence downstream of the gene, which is used in another step in the method of figure 4;
- Figure 4: is a representation of steps in the method of figure 1, including the steps of transfecting a cell with plasmids of figure 3 and ssRNA of figure 2, and including the testing of the rescued virus strain according to a preferred embodiment of the invention;
- Figure 5: is an illustration of the DNA sequence of the genome segment encoding VP6 of AHSV strain FR (Fourie) - the translational start codons in the NS4 open reading frame (ORF) are shown in grey;

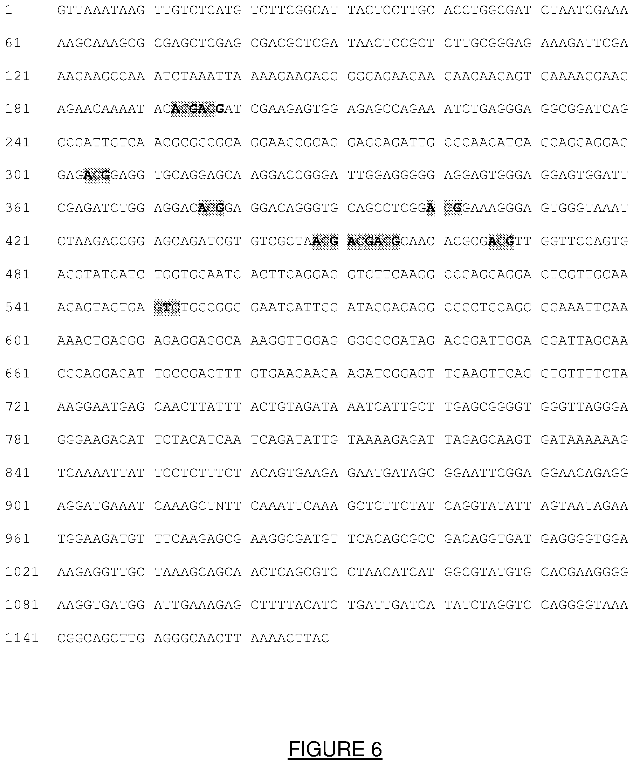

- Figure 6: is an illustration of the DNA sequence of genome segment encoding VP6 of FR lacking the translation start codons in the ORF of NS4
- Figure 7: is an illustration of the mapping of sequence reads obtained from the dsRNA purified from FR lacking NS4 against the wild-type sequence of the FR NS4 ORF proving that the recombinant virus lacks the translational start codons needed for NS4 expression;
- Figure 8: is a photograph of BSR cells infected with the vaccinal strain according to the invention illustrating the cytopathic effect (CPE) of the strain on the BSR cells;
- Figure 9: is a photograph of the purified genomic dsRNA of FR, lacking NS4 separated on a 1% agarose gel and stained with Ethidium Bromide;
- Figure 10: is a series of photographs of comparative immunoperoxidase monolayer assays (IPMA) of BSR cells distinguishing between cells that are infected with recombinant virulent AHSV called Fourie (FR) and vaccine virus of AHSV lacking the start codons in the NS4 ORF (FRΔNS4) according to the invention illustrating that there is no staining for AHSV NS4 in cells infected with FRΔNS4;
- Figure 11: is a photograph of FR (A) and FRΔNS4 (B) infected chicken embryos) after 3 to 6 days of infection, illustrating the avirulence of the vaccine according to the invention;
- Figure 12: is a picture showing results from semi-quantitative real-time RT-PCR of the embryos shown in figure 11, wherein the amplification curve of recombinant FR (A) shows that FR replicated well in the chicken embryos and that of FRΔNS4 (B) shows that FRΔNS4 did not replicate well in the chicken embryos which demonstrates that the innate immunity has stopped replication of the virus and that the vaccine according to the invention is non-pathogenic and replicates at low levels in innate immune competent hosts;
- Figure 13: is a graph showing results of real-time RT-PCR assaysof viraemia in horses after vaccination of horses with candidate vaccines and a control horse infected with recombinant virulent FR;
- Figure 14: is a graph showing measured rectal temperatures in horses vaccinated with candidate vaccines;
- Figure 15: provides photographs demonstrating the death of control horse 794 due to pulmonary oedema (evident from froth in the trachea) as a result infection with recombinant FR;
- Figure 16: is a graph showing the results of real-time RT-PCR measures of viraemia of horses after vaccination and challenge with an existing challenge virus for AHSV;
- Figure 17: is a graph showing the results from an AHSV VP7 blocking ELISA of horses vaccinated with the rAHSV4LP and rAHSV4 LPΔNS4 candidate vaccines; and
- Figure 18: is a graph showing body temperatures of horses after vaccination and challenge with an existing challenge virus for AHSV.

A preferred embodiment of the invention will now be described by way of example only with reference to the enclosed figures and photographs.

A method for producing a non-pathogenic live recombinant AHSV for use as a vaccine, according to a preferred embodiment of the invention includes the steps of:
- selecting a particular strain of AHSV;
- determining the genome sequence of the selected AHSV strain;
- altering the genome sequence of the AHSV strain such that the expression of the non-structural protein 4 (NS4) as a wild-type or complete protein is eliminated;
- synthesising transcription plasmids containing the altered genome sequence;
- synthesising expression plasmids containing codon optimised open reading frames of the proteins to provide a replication complex (VP1, VP3, VP4, VP6, VP7, and NS2);
- synthesising single stranded RNA (ssRNA) from the transcription plasmids;
- transfecting a permissive cell line with the said expression plasmids;
- subsequently transfecting the said transfected permissive cell line with ssRNA; and
- allowing the incorporation of the ssRNA into the replication complex in the transfected permissive cell line to produce replicating live attenuated recombinant AHSV.

The method of the invention thus provides a live attenuated recombinant vaccine virus of AHSV comprising a strain of AHSV of which the genome has been altered by substitution of at least one start codon of the open reading frame encoding for NS4 such that the expression of NS4 as a wild-type or complete protein has been eliminated and including a genome segment 2 encoding the serotype determining viral protein (VP2) and S6 (VP5) of another AHSV serotype.

It is foreseen that the above method according to the invention is to be applied in respect of all the serotypes of AHSV including AHSV serotype 1 to AHSV serotype 9 and already attenuated AHSV strains.

According to further preferred embodiments of the invention, synthetic ssRNA is produced for use in a method of transfecting a cell. The ssRNA is derived after *in vitro* synthesis from cDNA synthesised from and containing the genome sequence of the vaccine of AHSV according to the invention, wherein the ssRNA includes the genome of the said non-pathogenic strain that has been altered such as to eliminate the expression of NS4 as a wild-type or complete protein and including a genome segment 2 encoding the serotype determining viral protein (VP2) of another AHSV serotype.

A vaccine comprising the recombinant vaccine virus lacking expression of NS4 is prepared as exemplified below. The vaccine is preferably provided in the form of a multivalent vaccine, comprising a combination of the recombinant live vaccine of AHSV serotypes 1 to 9 prepared according to the method of the invention.

The vaccine is prepared in the form of an injectable preparation, including an injectable aqueous suspension. In use, the vaccine is administered in any one or more methods selected from the group consisting of subcutaneous, intramuscular, or intradermal.

The invention further relates to the use of the vaccine according to the invention in a method of vaccinating animals against AHS, the method including the step of delivering an effective amount of the vaccine according to the third aspect of the invention to the animal.

The amount of vaccine virus to be delivered to an animal can be determined using standard techniques; however, generally, the amount to be delivered should be in the range of 10,000 to 10,000,000 infectious units/ml.

The vaccine may optionally further include a pharmaceutically acceptable carrier, adjuvant or vehicle.

The pharmaceutically acceptable carrier is selected from the group consisting of water based adjuvant and liquid nano-particles containing an immune-stimulating compound and a combination thereof. Preferably, the pharmaceutical carrier is selected from SEPPIC MONTANIDE™ Pet Gel, SEPPIC MONTANIDE™ IMS 3012 and a combination thereof.

The invention further provides an immunisation kit comprising two containers each including at least four different serotypes of a recombinant AHSV according to the first aspect of the invention, the arrangement being such that the kit is suitable for use in a method of immunising an animal against all nine serotypes of AHSV.

It was found that African horsesickness virus encodes for a NS4 which displays characteristics illustrating that it is involved in virus pathogenesis. Reverse genetics allows the removal or replacement of the gene with a cloned version of the altered viral gene and generation of the resulting viral strain using virus permissive cells. Virus containing the desired modification is isolated by screening technologies well known to a person skilled in the art.

In this description, the term "vaccine virus" means a live viral strain that is suitable for being used in a vaccine for immunising the particular host that is normally affected by the wild-type virus. A vaccine virus is one that is non-pathogenic and therefore, it does not cause the disease that is normally associated with the wild-type virus. The concept of vaccine virus is well known to those skilled in the art. Virulent viruses can be attenuated or inactivated so that they generate an immune response in a host immunised with the attenuated or inactivated virus without causing clinical signs/pathology. This allows a host to mount an effective immune response if the host is exposed to the wild-type virus.

The term "serotyped" AHSV means a "synthetic" AHSV re-assortant with outer shell proteins from other AHSV serotypes. In AHSV, VP2 is one of the two proteins (with VP5) which constitute the virus particle outer capsid. VP2 is the principal serotype specific antigen and the major target for neutralising antibodies. In the present invention, for example, a AHSV serotype 4 can be "serotyped" to generate a set of nine individual "serotyped" single gene re-assortants by replacing genome segment 2 (S2) encoding the serotype determining VP2 proteins of each of the nine AHSV serotype, respectively. It was found that for some serotypes it is necessary to replace the matching segment 6 as well which encodes VP5.

In the present invention, the virus is selected from any of the nine AHSV serotypes, including the attenuated strains currently being used for vaccination. The present invention is intended to encompass any possible serotype of AHSV, known or as yet unknown, which falls within the definition of the AHSV categories.

Further in the present invention, the method for producing a recombinant vaccine virus further comprises propagating the recombinant vaccine virus in cell culture. Propagation of AHSV in cell culture is well known to those skilled in the art.

The term "backbone" specifies all the segments of AHSV but excluding genome S2 which encodes VP2 and/or S6 which encode VP2 and VP5 respectively.

The non-structural protein 4 (NS4) is encoded from an ORF present in the AHSV segments that also encode VP6.

In the present invention, the expression of NS4 as a wild-type or complete protein has been eliminated by removing all the start codons in the ORF encoding NS4 from the genome of AHSV, resulting in the virus not expressing NS4 . Consequently NS4 is not present in cells infected by the virus and the resulting vaccine virus is non-pathogenic. The expression of NS4 as a complete or wild-type protein is eliminated by introducing mutations into the NS4 open reading frame (ORF). The removal of all start codons in the NS4 ORF ensure that the virus does not revert back to a pathogenic phenotype. This also allows the use of techniques to differentiate between wild-type field virus and vaccine virus.

The alterations are introduced into the genome in any suitable way. For example, cDNA clones are made for the entire genome. Alterations are then introduced into the cDNA clone of the segment encoding VP6. Synthetic ssRNA transcripts from these cDNA clones are then used as the ssRNA to transfect a cell. In the present invention, the mutations are substitutions of all start codons (AUG) from the NS4 open reading frame, which inactivate the expression of NS4 but do not affect the reading frame or amino acid sequence of VP6.

The cells which are transfected with ssRNA are in the form of any cell which is suitable for being transfected with ssRNA and for culturing viral strains. For example, the cell used in transfection is a viral permissive cell. Preferably, the cell line is selected from the group consisting of but not limited to BHK 21 cells, Vero cells, 293T cells, BSR cells (a clone of BHK 21 cells), HeLa cells, C6/36 cells (a mosquito cell line derived from *Aedes albopictus*), or KC cells (a midge cell line derived from the insect vector *Culicoides sonorensis*). More preferably, the cells are BSR cells.

The ssRNA used to transfect the cell is a transcript of the genome of the virus and also comprises the mutations in the NS4 ORF. This transcript is obtained directly from *in vitro* transcripts made by virus cores which contain a mutation. Alternatively, the transcript is obtained indirectly from synthetically produced cDNA. In AHSV, viral ssRNA is synthesised in the cytoplasm of the infected cell by viral particles where it serves as mRNAs for viral protein synthesis and as a template for the synthesis of new genomic dsRNA. The ssRNA of the present invention also performs these functions and so the mutations in the ssRNA are incorporated into the vaccine virus during synthesis of the dsRNA genome.

It will be appreciated, however, that the altered genome segment could be incorporated in the AHSV in a number of methods which are known in the art including, but not limited to different steps such as:
- reproducing (NS4) altered virus ssRNA *in vitro* from purified virus cores and isolating the reproduced VP6 segment ssRNA;
- preparing cDNA of the altered (NS4) genome segment and cloning the same downstream from a T7 promoter and then reproducing the cDNA in vitro through T7 polymerase; and
- synthetically producing cDNA including the altered (NS4) genome and thereafter producing ssRNA synthetically from the cDNA, through T7 polymerase in T7 polymerase expressing cells or the like.

Numerous other methods or steps known in the art could be employed without deviating from the scope of this invention.

The transfected cell is cultured in any suitable way and under any suitable conditions to allow production of the vaccine virus, as is known in the art. Such methods of culturing cells are well known to those skilled in the art.

The step of transfecting the cell preferably comprises 2 or more transfection steps, wherein:
(1) a first transfection step comprises transfecting the cell with expression plasmids encoding six proteins required for the assembly of AHSV replication complexes; and
(2) a second transfection step comprises transfecting the above cell with ssRNA's which are transcripts of the viral genome or transcripts derived from transcription plasmids encoding viral genome and comprises the mutations, and therefore encodes all the components required for assembly and replication of the virus.

When working with AHSV, it is preferred that at least one of viral components VP2, VP5, NS1, NS3 and NS3a, is not encoded by the expression plasmids in the first transfection step. Preferably all of viral components VP2, VP5, NS1, NS3 and NS3a are omitted during the first transfection step. It is further preferred that there is a time gap between the first and second transfection steps so that there is sufficient time for protein expression and assembly of the viral replication complex. Preferably there is at least 6 hours, more preferably 12 hours and most preferably 24 hours between the first and second transfection steps.

Non-limiting examples of preferred embodiments of the invention are described in more detail below.

### WORKING EXAMPLES:

### EXAMPLE 1

The example which follows is given without any implied limitation, illustrates the present invention and shows how the invention may be carried out.

### MATERIAL AND METHODS

### Genome sequencing

The complete genome sequence of virulent AHSV serotype 5 known as Fourie (hereinafter "FR") was determined using new generation sequencing (NGS) after passage in suckling mice using the methods described in Potgieter *et al.,* (2009). (The sequence of FR is known (Potgieter *et al.,* 2015))

The complete genome sequence of virulent virus was determined from virus passaged only in suckling mice to preserve the pathogenicity of the virus.

### T7 plasmid clones used for the synthesis of authentic AHSV ssRNA.

cDNA clones of each of the genome segments of FR were synthesised and cloned by a commercial company called Genscript, which synthesises and clones the segments into the plasmid vector of choice.

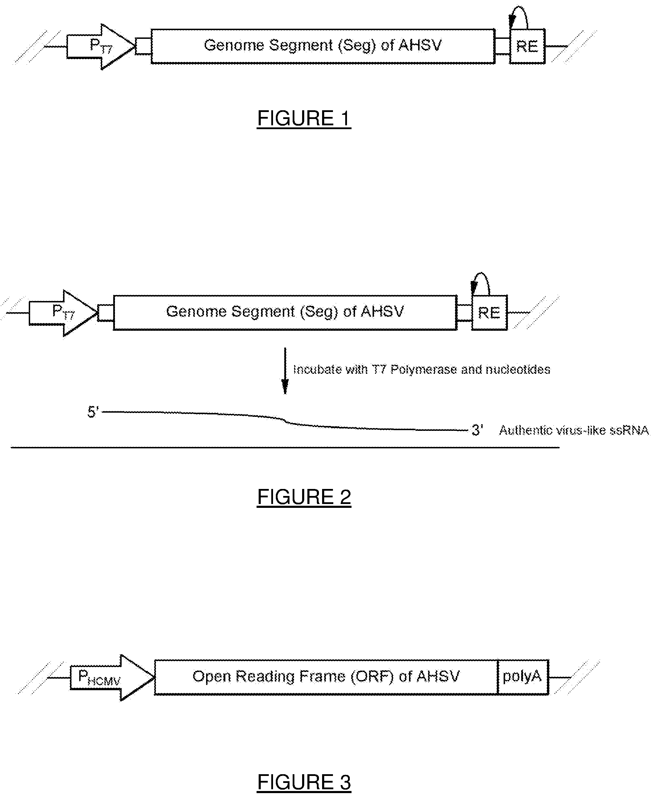

The cDNA of each genome segment of FR in each transcription plasmid was cloned directly downstream of a bacteriophage T7 DNA dependent RNA polymerase promoter sequence with a unique restriction enzyme site (for example *BsMBI*) directly downstream of the AHSV genome sequence (Figure 1). All T7 transcription plasmids from Genscript were transformed and propagated in *E.coli* DH5a or *E.coli* DH10B cells (Zymo research) according to manufacturer's instructions. Bacteria harbouring the transcription plasmids were propagated in Luria Broth containing 100µg/ml Carbenicillin and plasmids were purified using the Qiagen Qiaprep Spin Miniprep kit according to manufacturer's instructions.

### In vitro synthesis of AHSV transcripts from cDNA plasmid clones

T7 plasmid clones (transcription plasmids) were digested/linearised with restriction enzyme, BsMBI, BbsI or SapI, and digested plasmids were purified using a Qiagen Gel Extraction kit according to manufacturer's instructions. Single stranded RNA (ssRNA) transcripts with a 5' cap analogue (ARCA) were generated from the digested T7 plasmid clones using the mMessage mMachine T7 Ultra kit (AMBION) according to manufacturer's instructions (as illustrated in Figure 2). After ssRNA synthesis, DNA templates were removed using DNase and resulting ssRNA purified using the MEGAclear kit (AMBION) according to manufacturer's instructions. The concentration of ssRNA was determined using a Nanodrop spectrophotometer. The purified ssRNA of all ten segments were mixed in approximate equal molar amounts prior to transfection.

### Expression plasmids used for the generation of AHSV replication complexes

Expression plasmids that express the proteins that form the replication complex of AHSV were synthesised by a commercial company, Genscript at the instructions of the applicant. The expression plasmids will express the appropriate AHSV FR VP1, VP3, VP4, VP6, VP7 and NS2 proteins respectively. The expression plasmids contain the Cytomegalovirus (CMV) DNA dependent RNA polymerase II promoter upstream of the reading frame of each of the above mentioned AHSV genes with poly-T sequence downstream to facilitate translation of proteins (Figure 3). All expression plasmids received from Genscript were transformed and propagated in *E.coli* DH5a (Zymo research) according to the manufacturer's instructions. Expression plasmids were propagated on a large scale in LB (Luria Broth) containing 100 microgram per ml Carbenicillin and transfection grade expression plasmids were purified from these cells using Qiagen Endotoxin free kits (Qiagen) according to manufacturer's instructions. The concentration of plasmid DNA was determined using a Nanodrop spectrophotometer and prior to transfection, all six plasmids were mixed in approximately equal molar amounts.

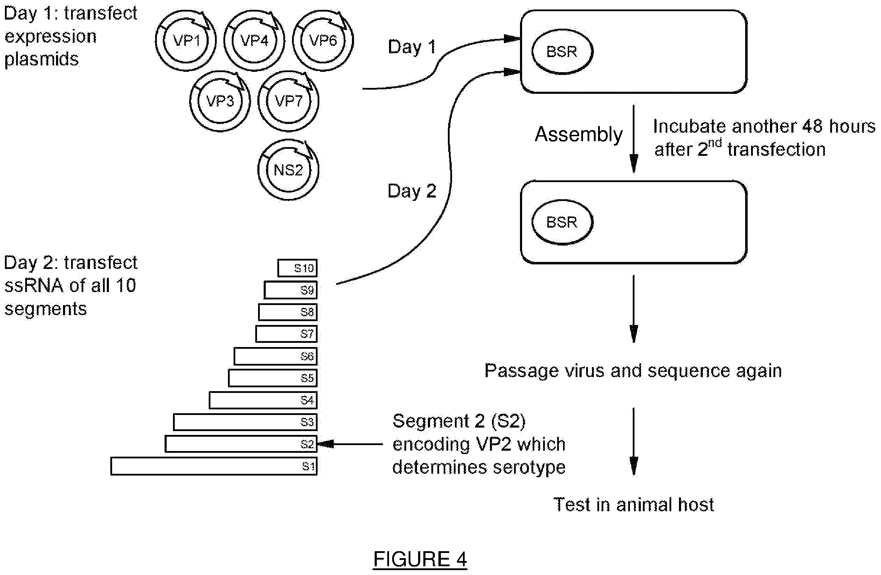

### Transfection of culture cells to recover FR from expression plasmids and ssRNA (as illustrated in Figure 4)

BSR cells were seeded in 24 wells in DMEM medium (Gibco) containing 5% FBS (Biochrom) and allowed to grow to 80% confluency. Prior to transfection the DMEM was removed and replaced with OPTI-MEM (Gibco). The cells were first transfected with 300ng of the mixture of expression plasmids (expressing VP1, VP3, VP4, VP6, VP7 and NS2) with Lipofectamine 2000 (Thermo Scientific) according to the manufacturer's prescription. At least 12 hours after the first transfection, the medium was changed with fresh OPTI-MEM and cells were transfected with 600nanograms of the mixture of the 10 ssRNA transcripts using the Lipofectamine 2000. At least 12 hours after the second transfection, the medium was changed to DMEM containing 5% FBS. Twenty-four hours after the second transfection, transfected cells were trypsinised (Trypsin from Gibco) and seeded into 25cm² flasks. The supernatant of the transfection was seeded onto fresh BSR cells grown to 90% confluency in a 25cm² tissue culture flask (Corning). The infected and trypsinised cells were incubated until cytopathic effects (CPE) were visible which indicates the successful rescue of virus. When CPE was observed, the virus was passed at least once more on BSR cells in a 75cm² tissue culture flask until CPE was complete (all cells detached from the surface of the tissue culture flask). Virus stocks were aliquoted in 1ml quantities and stored at -80 degrees C.

### Removal of start codon(s) from the ORF coding for NS4 in the VP6-seqment sequence

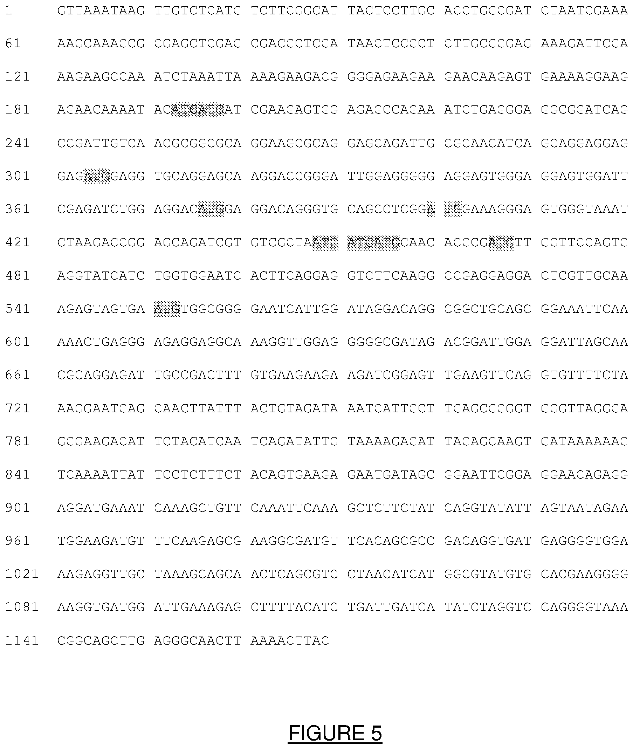

Sequence analysis of the gene encoding NS4 in the genome segment encoding for AHSV FR VP6 revealed that the open reading frame coding for NS4 contains a total of ten putative start codons (start codons, ATG, are highlighted in grey in Figure 5). To suppress or deactivate the expression of NS4 protein on the virus, all ten start codons (ATG) in the NS4 ORF were changed to ACG (9) or GTG (1). When RNA is transcribed from this DNA, there are no start codons (AUG's) in the ORF of NS4 and therefore no part of the NS4 protein can be translated. However, despite these changes the VP6 ORF is still intact and VP6 with normal amino acid sequence can be produced. Transcription plasmids containing genome segments having the modified NS4 ORF were also synthesised by Genscript. The modified NS4 plasmids were also transformed and propagated as described above. ssRNA of the segment containing mutations according to the invention was synthesised and purified as described above.

### Transfection of cultured cells to recover recombinant FR lacking NS4 protein (rFRANS4) from expression plasmid and ssRNA

rFRΔNS4 was recovered from expression plasmids and ssRNA transcripts as described above for the rescue of FR. However the T7 ssRNA transcript for the VP6 encoding segment contained mutations that removed all start codons in the NS4 open reading frame.

To confirm the identity of the rescued virus, viral dsRNA was purified from passaged BSR cells as described in Potgieter *et al* (2009) and separated on a 1% agarose gel.

### Confirmation of the lack of NS4 expression from FRΔNS4 using an immunoperoxidase monolayer assay (IPMA)

BSR cells were seeded in 96 well plates (4 rows for each virus). The cells were infected with dilutions of recombinant virulent FR, recombinant FR minus NS4 or no virus (mock infected). When cells infected with both viruses started showing CPE (foci), the medium was removed and cells were fixed with 4% paraformaldehyde for 10 minutes and washed with PBS (Gibco). Cells were then permeabilised with PBS-T (PBS containing 0.05% Tween 20), and blocked with 10% milkpowder (Elite) in PBS-T for 40 minutes. Then, rabbit anti-NS2 and rabbit anti-NS4 serums were added in 5% milkpowder/PBS-T at dilutions of 1:1000 and 1:400 anti NS2 and NS4 respectively. Following incubation at 37 degrees C for an hour, wells were washed 3 times with 300µl PBS-T. Then, 100µl goat anti-rabbit HRP conjugate (Thermo Scientific) was added in 5% milkpowder/PBS-T at a dilution of 1:5000 and plates were incubated for an hour at 37 degrees C. After another three washes 100µl AEC ready-to-use substrate (Thermo Scientific) was added to each well and the plate was incubated for 15 minutes until staining was clear. The substrate was removed and wells washed once more with PBS. Stained cells were viewed with a Zeiss AXIO vert.A1 light microscope and photographs of cells magnified at 600x were taken with a Zeiss digital camera. Images were processed with Zeiss Zen imaging software.

### Characterisation of the growth of rFR and rFRΔNS4 in embryonated chicken eggs

An important characteristic of a vaccine is that it should be unable to cause any clinical signs associated with the virulent virus in the host or a suitable small animal model. To evaluate the pathogenicity of rFR and rFRΔNS4, 13 day old embryonated chicken eggs were injected intravenously with a 1:10 dilution of recombinant FR and recombinant FRΔNS4, respectively. The infected eggs were then incubated at 32 degrees Celsius and monitored daily. After 6 days of infection, live embryos were euthanised by chilling at 4 degrees C for at least 5 hours. Together with embryos that died prior to the 6 days incubation, each embryo was removed from the egg and examined for typical lesions caused by virulent AHSV in chicken embryos, like bleeding and oedema .Photographs were taken using a normal digital camera. After photographs were taken, the embryos were homogenised with a mortar and pestle and viral load (RNA load) was determined using semi-quantitative real-time RT-PCR based on AHSV segment 5 (S5/NS1) in accordance with methods known in the art.

### RESULTS

### Rescue of rFR rFR min NS4 (rFRANS4) from expression plasmids and T7 RNA transcripts

The recovery of infectious AHSV from the combinations of expression plasmids and exact-copy ssRNA transcripts have been demonstrated before (Kaname *et al.,* 2013 and van de Water *et al.,* 2015). Using the transfection method herein, transfected cells were passaged 24 hours after the transfection with FR ssRNA (second transfection) and seeded onto a 25cm² flask of BSR cells. Seventy-two hours later clear CPE was observed (not shown).

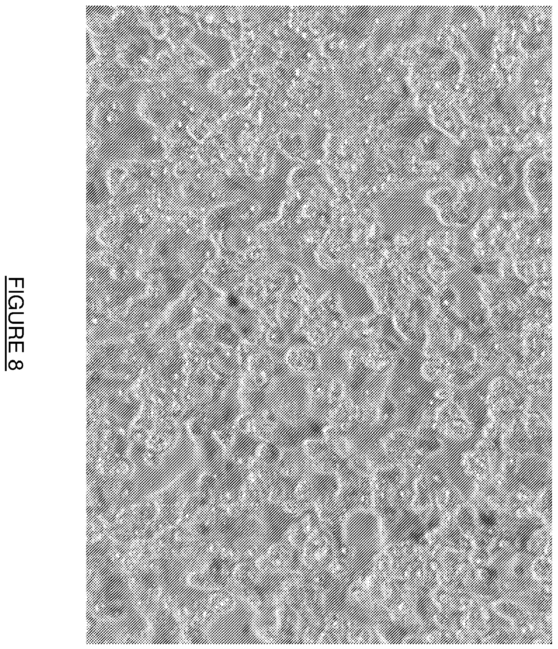

The recovery of rFRΔNS4 was performed with ssRNA transcripts containing the normal genome segments of FR but with exchange of the normal VP6 segment with the altered segment encoding VP6, but lacking start codons for NS4, comprising the mutations as described herein. The rescued virus was passaged as described herein and after 72 hours, clear CPE was observed. CPE for rFRΔNS4 initially developed slower compared to rFR. The titre of rFRΔNS4 at passage two in BSR cells was 10^{6.5} TCID₅₀/ml. The ability to propagate rFRΔNS4 in cell culture illustrate that the virus can be grown in large scale for vaccine production. Figure 8 shows the CPE observed on infected BSR cells infected with rFRΔNS4.

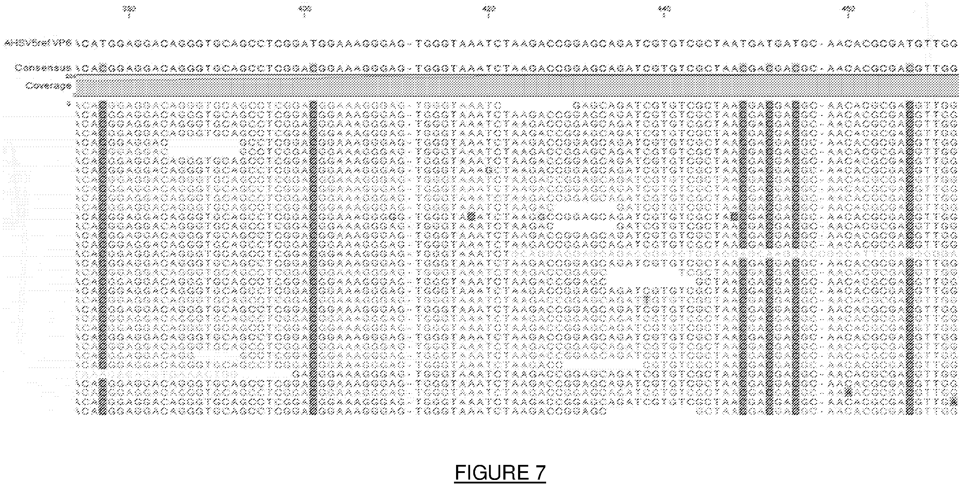

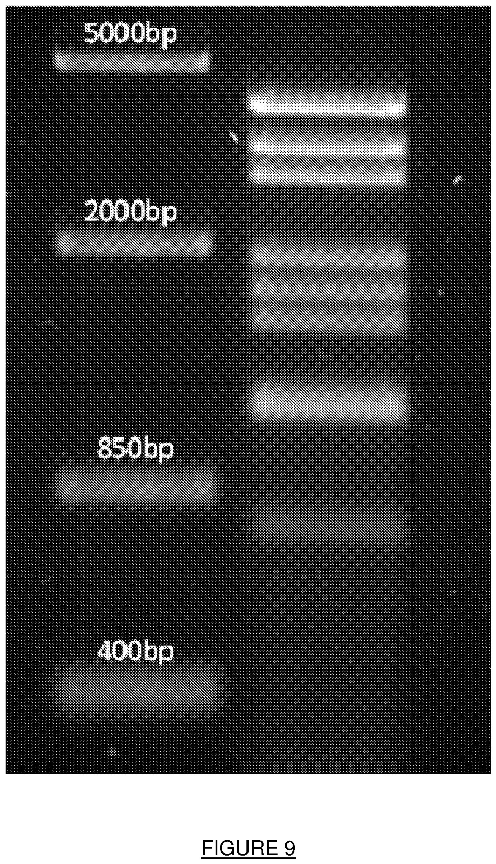

Genomic dsRNA from the rescued FRΔNS4 was extracted, purified and analysed on a 1% agarose gel as described in Potgieter *et al.,* (2009). The dsRNA of FRΔNS4 shows the typical size distribution of AHSV dsRNA on a 1% agarose gel which confirms that AHSV had been recovered (Figure 9). To confirm that the rescued FR and rFRΔNS4 had the correct genome sequences, the purified viral dsRNA was sequenced using New Generation sequencing (NGS) technology (MiSeq). After mapping of resulting sequences to wild-type FR sequence, the identity of FR was confirmed (not shown) and the presence of mutations in the VP6 encoding segment of rFRΔNS4 was also confirmed (Figure 7), demonstrating that the virus recovered contained the desired mutations. Figure 7 shows detailed results of the rFRΔNS4 VP6 coding segment sequence mapped against wild-type FR sequence in which some of the ATG to ACG mutations are clear.

### The use of IPMA to prove that rFRΔNS4 does not produce NS4

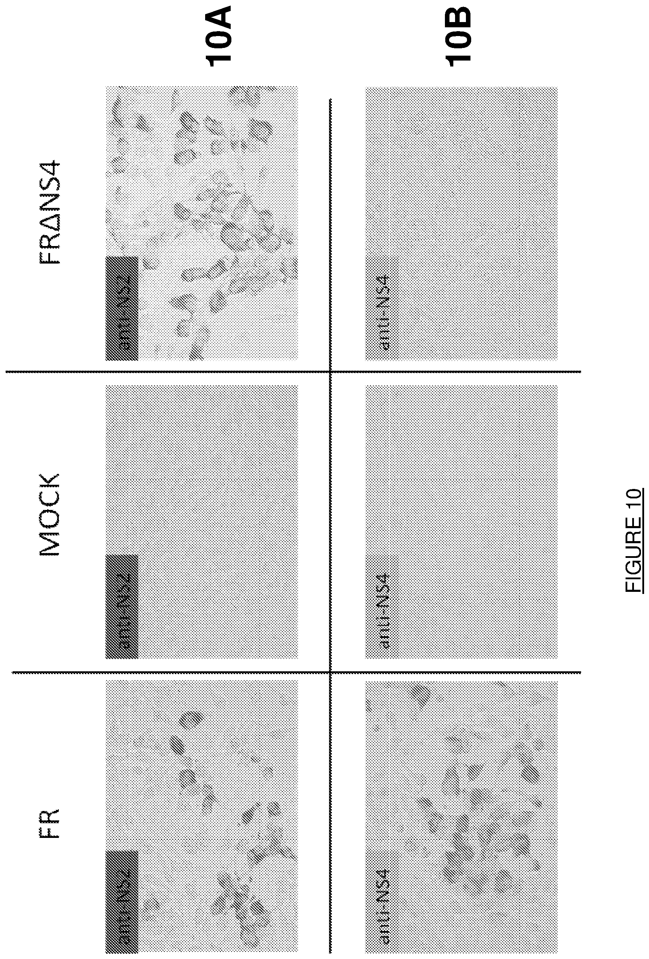

To confirm that rFRΔNS4 was not expressing NS4 , fresh BSR cells were seeded into a 96 wells plate and infected with both rFR and rFRΔNS4, respectively. When CPE developed, the cells were subjected to IPMA staining as described above. Figure 10(A) show clearly that both rFR and rFRΔNS4 produce AHSV protein NS2 but Figure 10(B) show that only rFR produces NS4. The specificity of the assays was demonstrated by the fact that uninfected cells show neither NS2 nor NS4 staining.

### The assessment of virulence replication in embryonated chicken eggs

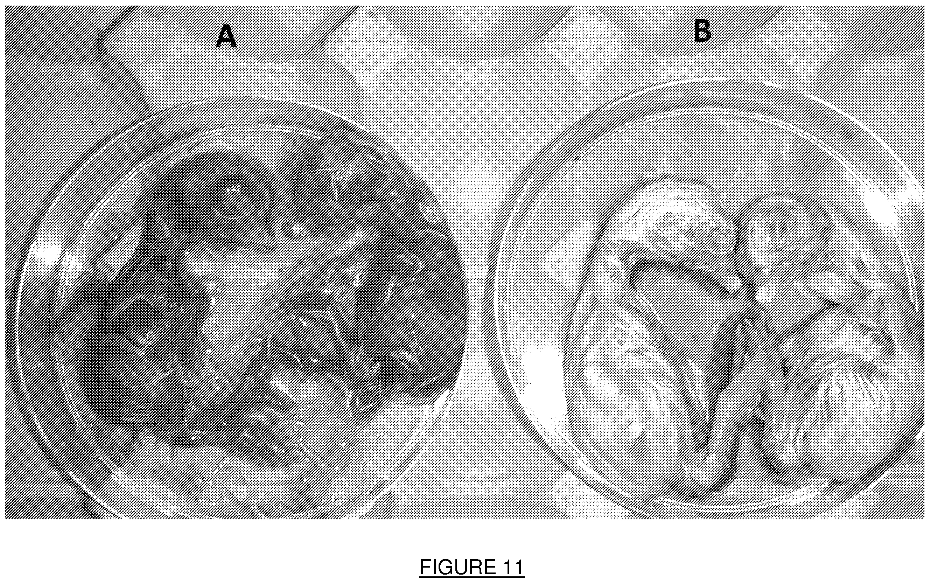

To evaluate whether or not rFRΔNS4 is pathogenic or attenuated, embryonated chicken eggs (ECE) were used as a small animal model. The use of embryonated chicken eggs in AHSV research is well established in the field and well known to the person skilled in the art (Maartens *et al* 2011). Thirteen day old embryonated eggs were infected with both rFR and rFRΔNS4, respectively and observed for 6 days. Infection of embryonated eggs with rFR led to the death of the embryo's 3 to 4 days after infection as expected after infection with virulent virus. Embryos infected with rFR also showed the typical signs of AHSV infection such as bleeding and oedema (Figure 11 (A)). In contrast rFRΔNS4 did not kill any of the infected eggs and the embryos did not show any of the signs of AHSV infection like haemorrhage and/or oedema (Figure 11 (B)).

### Characterisation of the growth of rFRΔNS4 in ECE

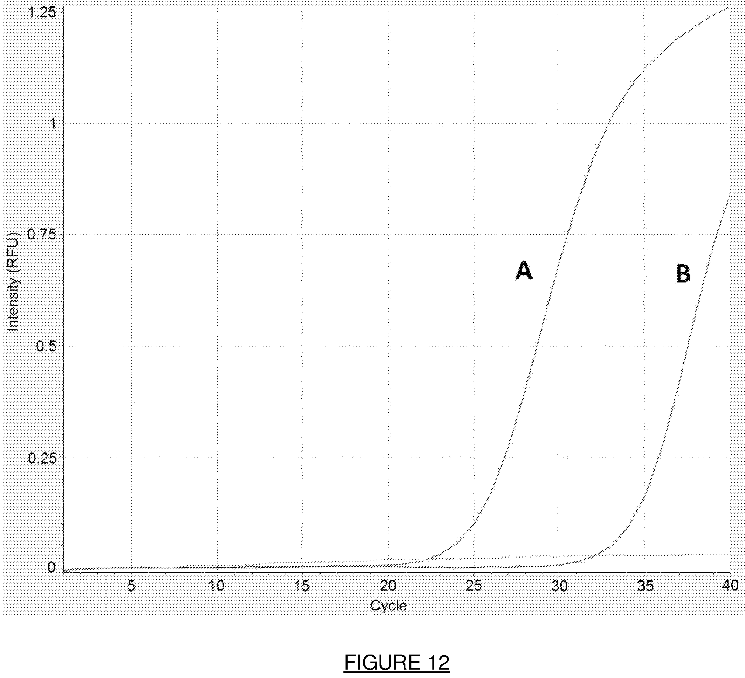

It is important to appreciate that rFRΔNS4 grows well in BSR cells but not in the small animal host. Semi-quantitative real-time RT-PCR based on AHSV segment 5 (S5/NS1) was performed to demonstrate the growth of both rFR and rFRΔNS4 in embryonated eggs. Figure 12 shows that rFR (amplification curve A) replicated well in the chicken embryos. In contrast rFRΔNS4 (amplification curve B) showed very limited levels of replication in chicken embryos. The substantial reduction in rFRΔNS4 replication compared to rFR, suggests that the innate immunity of the chickens eggs inhibited replication of rFRΔNS4.

### EXAMPLE 2

The example which follows is given without any implied limitation, illustrates the present invention and illustrates how the invention may be carried out.

### MATERIALS AND METHODS

### Horses

Horses of the "Boerperd" breed that tested susceptible to AHSV infection were used in all trials. Horses are from a farm in the Eastern Free-State Province of South Africa that does not experience yearly outbreaks of AHS and where horses are not vaccinated against AHS.

### Sampling

Blood in EDTA, Heparin and serum tubes (5ml) were taken daily for at least nine days after vaccination, seven days after booster and 14 days after challenge to monitor viraemia, do virus isolation and determine antibody titres. If horses showed any AHS clinical signs or had fever on days that were not part of planned daily bleeds, blood samples were taken on that day and a couple of days after until clinical signs/fever/viraemia subsided. Blood in EDTA was used for real-time RT-PCR, blood in Heparin for virus isolation and serum samples were used to determine levels of antibody using ELISA and virus neutralisation testing (VNT).

### Viruses

All viruses were rescued using reverse genetics using the methods described earlier. Both virulent AHSV5 (FR) described earlier as well as an attenuated AHSV4 (here called AHSV4LP) was rescued. The specific attenuated AHSV4 used here was the first AHSV strain attenuated by serial passage in cell culture (BHK21) and selection of large plaques in Vero cells (Erasmus 1973). The rescue of the attenuated virus was published by Van de Water *et al.,* 2015. Virulent and attenuated viruses with and without NS4 expression were rescued. Only one of the challenge viruses was not a recombinant virus (HS32/62). This virus has been used as challenge material for AHSV4 for many years (Erasmus, 1973).

### Challenge viruses and control horses

The challenge strain for AHSV4 vaccinated horses was HS32/62 - 2S - (Erasmus *et al.,* 1973). There was no control horse for AHSV4 since the challenge material has caused mortalities in all control horses in previous trials. The challenge virus for AHSV5 vaccinated horses was recombinant FR (rFR) described earlier. For ethical reasons only a single horse was used as unvaccinated control for AHSV5 and the same horse served to determine the virulence of recombinant FR (rFR).

### Vaccination

For each vaccine candidate two susceptible horses were injected. The numbers of the horses, vaccine candidates and challenge strains as well as the route of injection and virus titres are shown in table 1.

### AHSV VP7 blocking ELISA

To test the susceptibility of horses to AHSV infection and to determine when horses seroconverted after vaccination we used a commercial AHSV VP7 blocking ELISA from INGENASA according to the specifications of the manufacturer (Ingezim AHSV COMPAC PLUS 14.AHS.K3).

### Virus neutralisation tests

The following procedure was followed to determine the virus neutralising antibody titres in horse serums. Serial two fold dilutions of serum from vaccinated horses were made in the wells of a 96 well NUNC TC plate. Fifty µl of DMEM (1x) + Glutamax (Gibco) containing 1% Anti-Anti (Gibco), was added across the 96 well TC plates (NUNC). Hundred TCID50 of AHSV4 and five reference antigens were added to the serum dilutions on the plate, followed by an incubation step at 37°C for thirty minutes. Hundred µl of VERO cells in DMEM (1x) + Glutamax containing 5% FBS (Biochrom) were added to each well, before transferring the plates to a TC incubator containing 5% CO₂. Plates were incubated and evaluated daily for the progression of CPE until wells containing the working dilution of virus (100 TCID50) and negative serum showed 100% CPE.

Neutralization titres are expressed as the reciprocal of the dilution of serum that caused a 50 % inhibition of CPE.

### RNA extraction and real-time RT-PCR

Total nucleic acid was extracted from 100µl fresh horse blood in EDTA using the MagNAPure Compact robot and the MagNA Pure Compact Nucleic Acid Isolation Kit I. Nucleic acid was eluted in 100µl.

To determine viral load in horses after vaccination and challenge real-time RT-PCR targeting AHSV segment 5 (NS1) developed specifically for vaccine trials was used. Real-time RT-PCR reactions consisted of 2.1 µl water. 0.65µl Activator (Roche), 200nM of each primer and 100nM of Hydrolysis probe, 3.75µl of 2.7x RT-PCR mix (Roche LC480 RNA Master HybProbes) and 3µl of sample RNA. PCR reactions were cycled for 1 cycle at 98 degrees Celsius for 20 seconds (dsRNA denaturation), 1 cycle at 61 degrees Celsius for 5 minutes (Reverse transcription) and 40 cycles of 95 degrees Celsius for 10 seconds and 61 degrees Celsius for 20 seconds (target amplification and detection). Cycling and fluorescence detection were done in a LightCycler Nano real-time PCR machine (Roche) and LightCycler Nano software version 1.1 was used to analyse amplification data.

### RESULTS AND DISCUSSION

### Virulence of recombinant FR

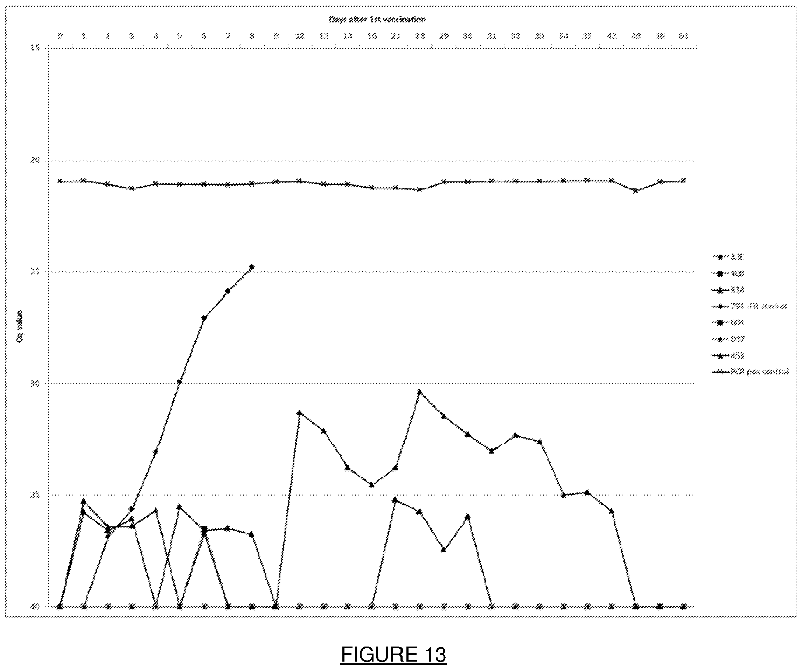

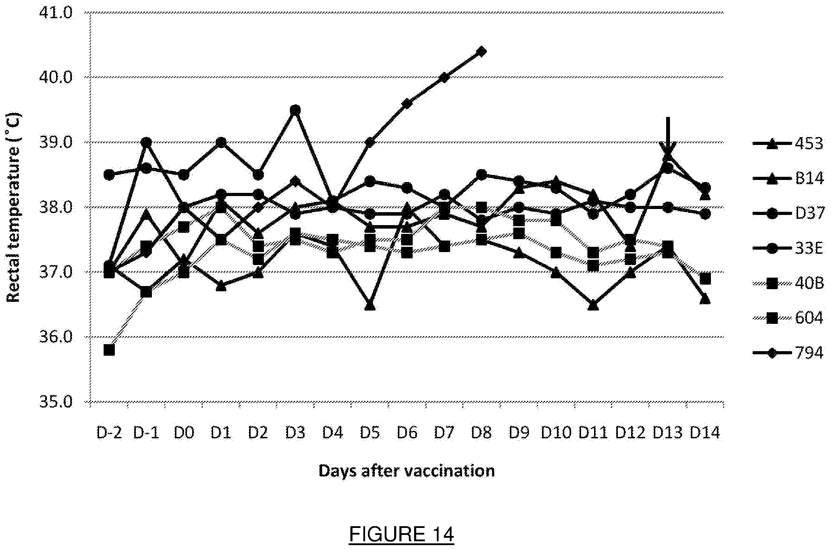

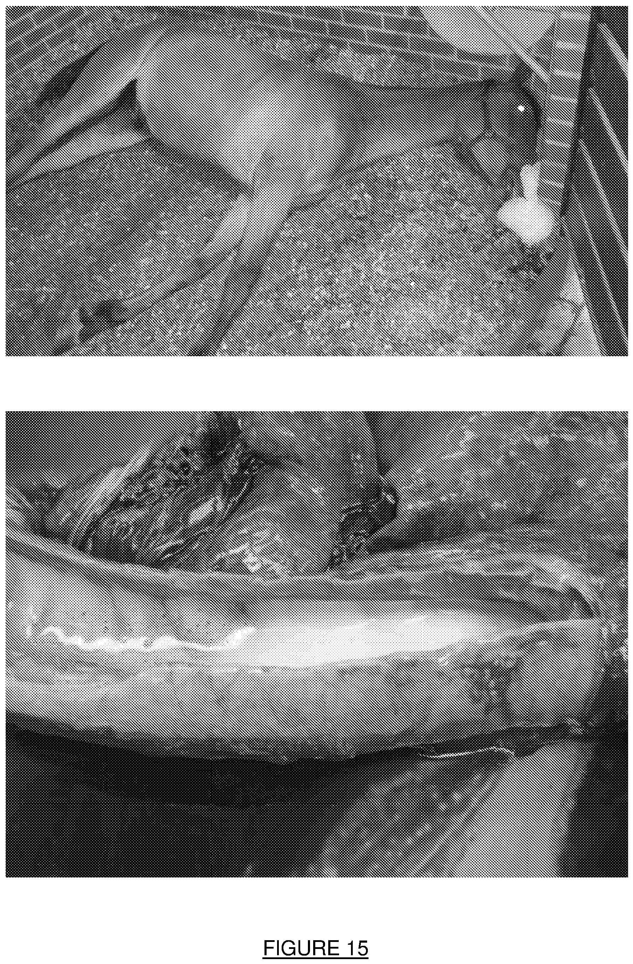

The control horse (794) that was infected with recombinant FR (rFR) showed viraemia from day 2 after challenge as measured using real-time RT-PCR (figure 13). The viraemia clearly increased daily and a minimum Cq of 24.8 was reached on day 8. A clear rise in the body temperature of horse 794 was evident from day 5 onwards which reached a maximum of 40.3 degrees C on the afternoon of the 8^{th} day (figure 14). On the morning of the 8^{th} day after challenge the horse showed laboured breathing and inappetence. The horse was found dead on the morning of the ninth day after challenge with foam exuding from its nostrils - it had probably expired the night before. Post mortem examination showed very severe pulmonary oedema. In addition to severe interstitial lung oedema, there was also clear alveolar oedema and foam in the trachea (figure 15). Mild hydrothorax and hydropericardium was also evident (not shown). These are typical post mortem lesions associated with the pulmonary form of AHS caused by virulent AHSV (Erasmus, 1973).

In addition to the control horse (794) another horse (623) that was vaccinated with an unsuccessful AHSV5 vaccine candidate and challenged with rFR developed serious disease and had to be euthanised. Also, rFR with genome segment 2 (S2) of a known virulent AHSV6 virus (HS39/63) was virulent in chicken embryos and a control horse (048) challenged with this virus died on the morning of the 7^{th} day after challenge showing clear signs of alveolar oedema. Post mortem examination of horses 623 and 048 revealed that both horses also had the pulmonary form of horsesickness (results not shown).

These results clearly indicate that rFR rescued using the genome sequences of FR, is completely virulent and a virus "serotyped" by exchange of only S2 was also virulent. Horse 794 also served as an unvaccinated control horse which validates the challenge of vaccinated horses with rFR.

### Virulence and immunogenicity of rFR lacking NS4 (rFRΔNS4)

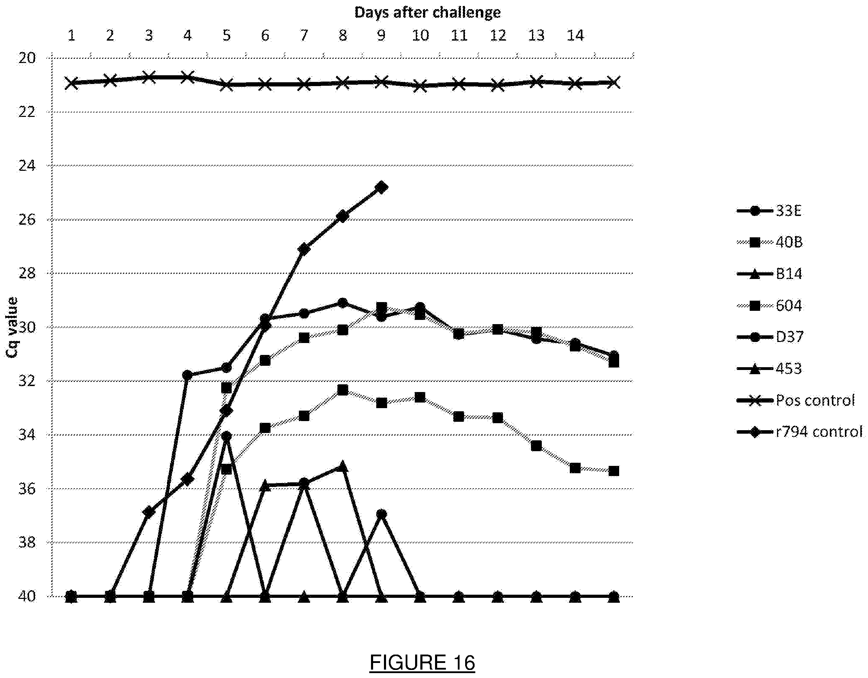

Neither of the horses injected with rFRΔNS4 showed clinical signs of AHS. Although viraemia could be detected in one of the two horses (453) the horse had mild pyrexia (39.2 °C on the afternoon of the 12^{th} day post infection). Compared to recombinant FR, rFRΔNS4 was attenuated as in the chicken embryo model. Both horses infected with rFRΔNS4 developed very high AHSV5 neutralising antibody titres on the 28^{th} day after the first injection (table 2). The horses had virus neutralising titres of higher than 256 until day 63. Eighty seven days after the first vaccination both horses vaccinated with rFRΔNS4 were challenged with rFR. Neither of the horses showed significant viraemia (Figure 16), neither had any clinical signs of AHS and both horses were protected against virulent challenge compared to the control unvaccinated horse (794) that showed high viraemia and died from the pulmonary form of AHSV.

### Virulence and immunogenicity of AHSV4LP and AHSV4LPminNS4

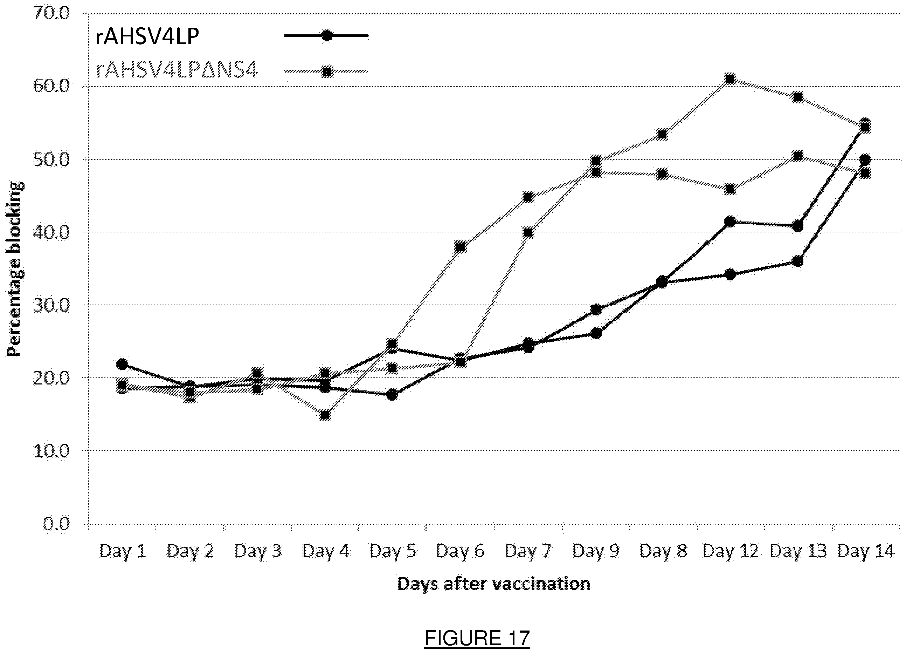

Two horses each were also injected with recombinant attenuated AHSV4 and the same virus lacking NS4 expression (here called rAHSV4LP and rAHSV4LPΔNS4). The horses vaccinated with rAHSV4LP did not receive a booster vaccination since we needed to find out whether this recombinant virus behaved the same as the original AHSV4 LP vaccine described by Erasmus (1973). The horses vaccinated with rAHSV4LPΔNS4 received a booster vaccination on day 28 after the first vaccination since it was hypothesised that removal of NS4 expression from an already attenuated virus may over-attenuate the virus. None of the horses vaccinated with these viruses showed any viraemia or fever over the period assessed (Figures 13 and 14) except for horse D37 which had a high temperature on the day of vaccination which was probably due to an equine herpes virus infection. Interestingly, horses vaccinated with rAHSV4LPΔNS4 seroconverted 5 days earlier than those vaccinated with rAHSV4LP (Figure 17). This result clearly indicates that AHSV NS4 inhibits the horse immune system. The virus neutralising titre induced by both AHSV4LP and AHSV4LPminNS4 (table 2) was sufficiently high to warrant challenge of the horses with virulent AHSV4 (Table 2). After challenge, one horse vaccinated with rAHSV4LP showed viraemia and had mild fever for a brief period (day 4 after vaccination). This coincided with the increase in viraemia (figure 16). Despite the viraemia and slight increase in body temperature for one day, the horse showed no clinical signs associated with AHS. Horse D37 vaccinated with AHSV4LP was fully protected against AHS. This makes sense when the virus neutralising titre is taken into account.

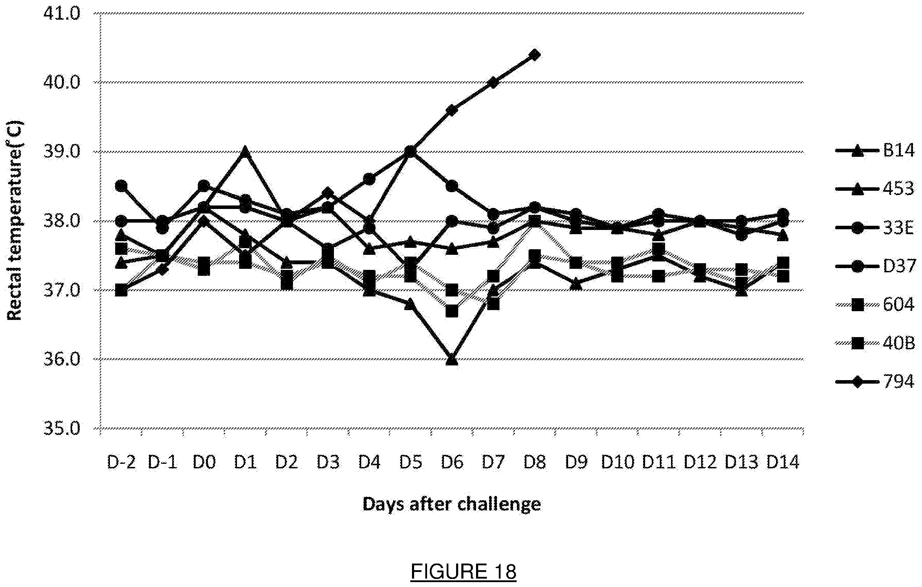

Both horses vaccinated with rAHSV4LPΔNS4 showed significant virus replication after challenge (figure 16) indicating the removal of NS4 expression from AHSV4LP may have over-attenuated the virus. Despite the virus replication, neither of the animals had any fever or any signs of AHS compared to horse 33E that had fever with similar levels of virus replication (figure 18). The absence of fever in these animals is interesting given the fact that both horses vaccinated with rAHSV4LPΔNS4 showed replication of challenge virus and especially since horse 604 had similar levels of virus replication compared to horse 33E (figure 16). Whether this has something to do with absence of NS4 in the vaccine strain is not known. Other horses that were challenged with the same virus in another trial and which showed similar virus replication also showed fever and some even clinical signs of AHS such as swollen supra-orbital fossae (results not shown).

From the above it was concluded that:
- the recombinant FR (rFR) is virulent;
- the recombinant FR lacking NS4 expression (rFRANS4) according to the invention is attenuated;
- rFRΔNS4 is highly immunogenic - it is more immunogenic than the well characterised AHSV4 LP;
- rAHSV4LP is attenuated and induces immunity similar to wild-type AHSV4LP;
- rAHSV4LPΔNS4 induces sero-conversion against AHSV VP7 up to five days faster than normal rAHSV4LP;
- rAHSV4LPΔNS4 induces full protection against AHS clinical signs including pyrexia despite virus replication; and
- virulent virus lacking NS4 confers complete protection against virulent challenge.

The recovery of AHSV from synthetic RNA transcripts allows for generation of mutants with consistent background. Using the established method of AHSV recovery from protein-expressing plasmids and ssRNA transcripts, the present invention described herein a modified virulent AHSV strain lacking NS4 expression. The AHSV lacking NS4 expression according to the invention grows well in BSR cells but does not replicate well in chicken embryos or horses. The ability to grow the novel AHSV strain in BSR cells shows that production of the virus will be as easy as the current live vaccines used in South Africa.

A single backbone lacking NS4 is used to prepare vaccines for all serotypes which are stable and safe. It was found that the virus according to the invention has a relatively low level of replication in live animals thus limiting the chances of the virus reverting to virulence through re-assortment with virulent strains. Owing to the fact that the same backbone is used in respect of different serotypes, the replication of the vaccine virus would be at the same rate for different serotypes unlike current live vaccines where the different serotypes replicate at different levels in horses. This further increases the stability and safety of the vaccine and in particular combination vaccines.

In one embodiment, up to five vaccines according to the invention for different serotypes could be combined in a single dosage. By preparing two vials, the one containing vaccines according to the invention for five serotypes and the other for the remaining serotypes, an animal could be immunised against all serotypes by two dosages administered concomitantly.

It was found that a vaccine according to the invention is effective in soliciting an effective immune response in animals against AHSV.

It was further found that since alterations have been made in the genome, it is possible to distinguish the virus according to the invention from field viruses through PCR and/or sequencing techniques.

It will be appreciated that variations in detail are possible without departing from the scope of the appended claims.

**Table 1. Horse numbers, viruses used and virus titres**

| **Horse number** | **Vaccine and route of 1^{st} vaccination and virus titre** | **Vaccine and route of second vaccination and virus titre** | **Challenge virus (all IV route)** |
|---|---|---|---|
| **D37** | rAHSV4LP- IM (10^{8.6} TCID50) | - | HS32/62-2S (10^{6.4} TCID50) |
| **33E** | rAHSV4LP- IM (10^{8.6} TCID50) | - | HS32/62- 2S (10^{6.4} TCID50) |
| **604** | rAHSV4LP ΔNS4 - IM(10^{7.8} TCID50) | rAHSV4LP ΔNS4-IM(10^{7.8} TCID50) | HS32/62- 2S (10^{6.4} TCID50) |
| **40B** | rAHSV4LP ΔNS4-IM(10^{7.8}TCID50) | rAHSV4LP ΔNS4-IM(10^{7.8}TCID50) | HS32/62- 2S (10^{6.4} TCID50) |
| **453** | rFRANS4-IV (10^{6.8} TCID50) | - | rFR (794 lung-1S) (10⁵⁻³ TCID50) |
| **B14** | rFRANS4-IV (10^{6.8} TCID50) | rFRΔNS4-IM (10^{6.8} TCID50) | rFR (794 lung-1 S) (10^{5.3} TCID50) |
| **794** | | - | rFR (#2 BSR from rescue) (10^{5.8} TCID50) |

| | | | |
|---|---|---|---|
| IM - Intramuscular IV - Intravenous 1S - 1 passage in suckling mice | | | |

Titres were determined on BSR cells

**Table 2. Virus neutralising antibody titres in horses after vaccination.**

| | | **Virus neutralising titre** | | |
|---|---|---|---|---|
| **Horse nr:** | **Vaccinated with:** | **Day 21** | **Day 28** | **Day 63** |
| **33E** | rAHSV4LP | 32 | 32 | 64 |
| **D37** | | 64 | 128 | 128 |
| **40B** | rAHSV4LPΔNS4 | 32 | 64 | 128 |
| **604** | | 32 | 32 | 128 |
| **B14** | FRΔNS4 | 8 | 1024 | ≥ 256 |
| **453** | | 8 | ≥ 4096 | ≥ 256 |

### REFERENCES

BELHOUCHET M, MOHD JAAFAR F, FIRTH AE, GRIMES JM, MERTENS PP et al., 2011 DETECTION OF A FOURTH ORBIVIRUS NON-STRUCTURAL PROTEIN. PLOS ONE 6: E25697.
BURRAGE TG, TREVEJO R, STONE-MARSCHAT M, LAEGREID WW. 1993. NEU-TRALIZING EPITOPES OF AFRICAN HORSESICKNESS VIRUS SEROTYPE 4 ARE LOCATED ON VP2. VIROLOGY 196:799-803.
ERASMUS BJ. 1973. THE PATHOGENESIS OF AFRICAN HORSESICKNESS, P. 1-11. IN BRYANS JT, GERBER H (ED), PROCEEDINGS OF THE THIRD INTERNATIONAL CONFERENCE ON EQUINE INFECTIOUS DISEASES, VOL. III. KARGER, BASEL, SWITZERLAND.
FIRTH AE. 2008. BIOINFORMATIC ANALYSIS SUGGESTS THAT THE ORBIVIRUS VP6 CISTRON ENCODES AN OVERLAPPING GENE. VIROL. J. 5:48.
KANAME Y, CELMA CC, KANAI Y, ROY P. RECOVERY OF AFRICAN HORSE SICKNESS VIRUS FROM SYNTHETIC RNA. J GEN VIROL. 2013 OCT;94(PT 10):2259-65. DOI: 10.1099/VIR.0.055905-0. EPUB 2013 JUL 16.
MAARTENS LH, ERASMUS BJ, CLIFT SJ. 2011. TISSUE TROPISM OF AFRICAN HORSESICKNESS VIRUS IN THE CHICKEN EMBRYO DEMONSTRATED WITH THE AVIDIN-BIOTIN COMPLEX IMMUNOPEROXIDASE METHOD. VETERINARY PATHOLOGY. 48(6):1085-1093.
MELLOR PS, AND BOORMAN J 1995. THE TRANSMISSION AND GEOGRAPHICAL SPREAD OF AFRICAN HORSE SICKNESS AND BLUETONGUE VIRUSES. ANN TROP MED PARASITOL. 89: 1-15.
MCINTOSH BM 1958. IMMUNOLOGICAL TYPES OF HORSESICKNESS VIRUS AND THEIR SIGNIfiCANCE IN IMMUNIZATION. ONDERSTEPOORT J. VET. RES. 27:465-538.
OURA CA, IVENS PA, BACHANEK-BANKOWSKA K, BIN-TARIF A, JALLOW DB ET AL., 2011. AFRICAN HORSE SICKNESS IN THE GAMBIA: CIRCULATION OF A LIVE-ATTENUATED VACCINE-DERIVED STRAIN. EPIDEMIOL INFECT: 1-4.
PAWESKA, JT, PRINSLOO S AND VENTER, GJ 2003. ORAL SUSCEPTIBILITY OF SOUTH AFRICAN CULICOIDES SPECIES TO LIVE-ATTENUATED SEROTYPE-SPECIFIC VACCINE STRAINS OF AFRICAN HORSE SICKNESS VIRUS (AHSV). MED VET ENTOMOL 17: 436-447.
POTGIETER AC, PAGE NA, LIEBENBERG J, WRIGHT IM, LANDT O, VAN DIJK AA. 2009. IMPROVED STRATEGIES FOR SEQUENCE-INDEPENDENT AMPLIFICATION AND SEQUENCING OF VIRAL DOUBLE-STRANDED RNA GENOMES. J GEN VIROL 90:1423-1432.
POTGIETER AC, WRIGHT IM, VAN DIJK AA. 2015. CONSENSUS SEQUENCE OF 27 AFRICAN HORSE SICKNESS VIRUS GENOMES FROM VIRUSES COLLECTED OVER A 76-YEAR PERIOD (1933 TO 2009). GENOME ANNOUNC 3(5):E00921-15.
RATINIER, M, CAPORALE M, GOLDER M, FRANZONI G, ALLAN K et al., 2011. IDENTIFICATION AND CHARACTERIZATION OF A NOVEL NON-STRUCTURAL PROTEIN OF BLUETONGUE VIRUS. PLOS PATHOG 7: E1002477.
ROY P, MERTENS PP, CASAL I. 1994. AFRICAN HORSESICKNESS VIRUS STRUCTURE. COMP. IMMUNOL. MICROBIOL. INFECT. DIS. 17:243-273.
TURNBULL, PJ, CORMACK SB AND HUISMANS H, 1996 CHARACTERIZATION OF THE GENE ENCODING CORE PROTEIN VP6 OF TWO AFRICAN HORSESICKNESS VIRUS SEROTYPES. J GEN VIROL 77(PT7): 1421-1423.
VAN DE WATER SGP, VAN GENNIP RGP, POTGIETER CA, WRIGHT IM, VAN RIJN PA. 2015. VP2 EXCHANGE AND NS3/NS3A DELETION IN AFRICAN HORSE SICKNESS VIRUS (AHSV) IN DEVELOPMENT OF DISABLED INFECTIOUS SINGLE ANIMAL VACCINE CANDIDATES FOR AHSV. J VIROL 89:8764 -8772.
ZWART L, POTGIETER CA, CLIFT SJ, VAN STADEN V (2015) CHARACTERISING NON-STRUCTURAL PROTEIN NS4 OF AFRICAN HORSE SICKNESS VIRUS. PLOS ONE 10(4): E0124281.

## Claims

1. A live attenuated recombinant African horsesickness virus (AHSV) of which the genome has been altered such that the expression of the non-structural protein 4 (NS4) as a wild-type or complete protein is eliminated.

2. The live attenuated recombinant AHSV of claim 1, wherein the recombinant virus has been altered such that the expression of NS4 as a wild-type or complete protein has been eliminated by substitution of at least one start codon of the open reading frame encoding for NS4, without affecting the functioning of virus protein 6 (VP6) of the virus.

3. The live attenuated recombinant AHSV of claim 2, wherein the number of start codons eliminated by substitution is from one to ten.

4. The live attenuated recombinant AHSV of claim 1, wherein the recombinant virus has been altered such that the expression of NS4 as a wild-type or complete protein has been eliminated by deletion of at least one start codon of the open reading frame encoding for NS4, without affecting the functioning of virus protein 6 (VP6) of the virus.

5. The live attenuated recombinant AHSV of claim 4, wherein the number of start codons eliminated by deletion is from one to ten.

6. The live attenuated recombinant AHSV of any of claims 1 to 5, wherein the AHSV is selected from the group consisting of AHSV serotype 1 to AHSV serotype 9, and attenuated AHSV strains.

7. The live attenuated recombinant AHSV of any of claims 1 to 6, wherein the recombinant virus includes a genome segment 2 and/or 6 encoding the serotype determining viral proteins (VP2 and 5) of another AHSV serotype or strain.

8. A method for producing a non-pathogenic live attenuated recombinant AHSV, the method including the steps of:
- selecting a particular strain of AHSV;
- determining the genome sequence of the selected AHSV strain;
- altering the genome sequence of the AHSV strain such that the expression of the non-structural protein 4 (NS4) as a wild-type or complete protein is eliminated;
- synthesising transcription plasmids containing the altered genome sequence;
- synthesising expression plasmids containing codon optimised open reading frames of the proteins to provide a replication complex;
- synthesising single stranded RNA (ssRNA) from the said transcription plasmids;
- transfecting a permissive cell line with the said expression plasmids;
- subsequently transfecting the said transfected permissive cell line with ssRNA; and
- allowing the incorporation of the ssRNA into the replication complex in the transfected permissive cell line to produce replicating live attenuated recombinant AHSV.

9. The method of claim 8, including the further step of re-sequencing the genome of the produced recombinant AHSV to confirm that the alteration to the genome has been effected.

10. The method of either claim 8 or claim 9, including the further step of replacing the synthetic ssRNA of the genome segment 2 encoding the serotype determining viral protein (VP2) with that of another AHSV serotype.

11. A vaccine including the live attenuated recombinant AHSV of any of claims 1 to 7.

12. The vaccine of claim 11 including a pharmaceutically acceptable carrier, adjuvant or vehicle.

13. The vaccine of claim 12, wherein the pharmaceutically acceptable carrier is selected from the group consisting of water based adjuvant and liquid nano-particles containing an immune-stimulating compound and a combination thereof.

14. A multivalent vaccine, the vaccine including a live attenuated recombinant AHSV of one serotype, as claimed in any of claims 1 to 7, and at least one other serotype of live attenuated recombinant AHSV, as claimed in any of claims 1 to 7.

15. Vaccine as described in any of claims 11 to 14, for use in vaccinating animals against AHSV.

16. An immunisation kit comprising two containers, each including at least four different serotypes of recombinant AHSV as claimed in any of claims 1 to 7, the arrangement being such that the kit is suitable for use in a method of immunising an animal against all nine serotypes of AHSV.

17. Vaccine as described in any of claims 11 to 14, for use in vaccinating animals against AHSV, the use including the step of delivering an effective amount of the vaccine as claimed in any of claims 11 to 14 to an animal in need thereof.

## Patentansprüche

1. Lebend attenuierter rekombinanter *African Horsesickness Virus* (AHSV, afrikanische Pferdepest), dessen Genom derart verändert ist, dass die Expression des Nichtstrukturproteins 4 (*non-structural protein 4,* NS4) als Wildtyp oder vollständiges Protein abgeschaltet ist.

2. Lebend attenuierter AHSV gemäß Anspruch 1, wobei der rekombinante Virus derart verändert ist, dass die Expression von NS4 als Wildtyp oder vollständiges Protein durch Substitution mindestens eines Startcodons des *Open Reading Frame* (ORF), kodierend für NS4, abgeschaltet ist, ohne dass die Funktionalität des Virusprotein 6 (VP6) des Virus beeinflusst wird.

3. Lebend attenuierter rekombinanter AHSV gemäß Anspruch 2, wobei die Anzahl der durch Substitution abgeschalteten Startcodons zwischen eins und zehn liegt.

4. Lebend attenuierter rekombinanter AHSV gemäß Anspruch 1, wobei der rekombinante Virus derart verändert ist, dass eine Expression von NS4 als Wildtyp oder vollständiges Protein durch Deletion mindestens eines Startcodons des *Open Reading Frame* (ORF), kodierend für NS4, abgeschaltet ist, ohne dass die Funktionalität des Virusproteins 6 (VPS) des Virus beeinflusst wird.

5. Lebend attenuierter rekombinanter AHSV gemäß Anspruch 4, wobei die Anzahl der durch Deletion abgeschalteten Startcodons zwischen eins und zehn liegt.

6. Lebend attenuierter rekombinanter AHSV gemäß einem der Ansprüche 1 bis 5, wobei der AHSV aus der Gruppe bestehend aus AHSV Serotyp 1 bis AHSV Serotyp 9 und attenuierten AHSV-Stämmen ausgewählt ist.

7. Lebend attenuierter rekombinanter AHSV gemäß einem der Ansprüche 1 bis 6, wobei der rekombinante Virus ein Genom-Segment 2 und/oder 6 umfasst, welche für den Serotyp bestimmende virale Proteine (VP2 und 5) eines anderen AHSV Serotyps oder Stamms kodieren.

8. Verfahren zur Herstellung eines apathogenen lebend attenuierten rekombinanten AHSV, wobei das Verfahren die nachfolgenden Schritte aufweist:
- Auswahl eines bestimmten Stamms von AHSV;
- Bestimmung der genomischen Sequenz des ausgewählten AHSV-Stamms;
- Veränderung der Genom-Sequenz des AHSV-Stamms derart, dass die Expression des Nichtstrukturproteins 4 (NS4) als Wildtyp oder vollständiges Protein abgeschaltet ist;
- Synthetisierung von Transkriptionsplasmiden, aufweisend die veränderte Genom-Sequenz;
- Synthetisierung von Expressionsplasmiden, aufweisend kodonoptimierte *Open Reading Frames* (ORF) der zur Bereitstellung eines Replikationskomplex erforderlichen Proteine;
- Synthetisierung einer einzelsträngigen RNA (ssRNA) von den vorgenannten Transkriptionsplasmiden;
- Transfektion einer permissiven Zelllinie mit den vorgenannten Expressionsplasmiden;
- nachfolgende Transfektion der vorgenannten transfektierten permissiven Zelllinie mit ssRNA; und
- Ermöglichen der Inkorporation der ssRNA in den Replikationskomplex in die transfektierte permissive Zelllinie, um replizierenden lebend attenuierten rekombinanten AHSV zu produzieren.

9. Verfahren gemäß Anspruch 8, umfassend den weiteren Schritt der Resequenzierung des Genoms des produzierten rekombinanten AHSV zur Bestätigung der Veränderung des Genoms.

10. Verfahren gemäß Anspruch 8 oder Anspruch 9, umfassend den weiteren Schritt des Ersetzens der synthetischen ssRNA des Genom-Segments 2, kodierend für das den Serotyp bestimmende virale Protein (VP2), mit dem eines anderen AHSV Serotyps.

11. Impfstoff, umfassend den lebend attenuierten rekombinanten AHSV gemäß einem der Ansprüche 1 bis 7.

12. Impfstoff nach Anspruch 11, umfassend einen pharmazeutisch verträglichen Träger, Hilfsstoff oder Trägerstoff.

13. Impfstoff nach Anspruch 12, wobei der pharmazeutisch verträgliche Träger ausgewählt ist aus der Gruppe bestehend aus wasser-basierten Hilfsstoffen und flüssigen Nanopartikeln, aufweisend eine immunstimulierende Komponente sowie deren Kombinationen.

14. Mehrwertiger Impfstoff, wobei der Impfstoff einen lebend attenuierten rekombinanten AHSV eines Serotyps aufweist, wie in einem der Ansprüche 1 bis 7 beansprucht, und mindestens einen weiteren Serotyp von lebend attenuierten rekombinantem AHSV, wie in einem der Ansprüche 1 bis 7 beansprucht.

15. Impfstoff nach einem der Ansprüche 11 bis 14 zur Verwendung in der Immunisierung von Tieren gegen AHSV.

16. Immunisierungs-Kit, aufweisend zwei Behälter, jeweils umfassend mindestens vier verschiedene Serotypen von rekombinanten AHSV, wie in einem der Ansprüche 1 bis 7 beansprucht, wobei die Ausgestaltung derart ist, dass das Kit zur Verwendung in einem Verfahren zur Immunisierung eines Tieres gegen alle neun Serotypen von AHSV geeignet ist.

17. Impfstoff gemäß einem der Ansprüche 11 bis 14 zur Verwendung in der Immunisierung von Tieren gegen AHSV, wobei die Verwendung den Schritt des Zuführens einer wirksamen Menge des Impfstoffs nach einem der Ansprüche 11 bis 14 dem zu behandelnden Tier umfasst.

## Revendications

1. Virus de la peste équine (AHSV) recombinant atténué vivant dont le génome a été altéré de sorte que l'expression de la protéine non structurelle 4 (NS4) en tant que type sauvage ou protéine complète est éliminée.

2. AHSV recombinant atténué vivant selon la revendication 1, dans lequel le virus recombinant a été altéré de sorte que l'expression de NS4 en tant que type sauvage ou protéine complète a été éliminée par substitution d'au moins un codon d'initiation du cadre de lecture ouvert codant pour NS4, sans affecter le fonctionnement de la protéine virale 6 (VP6) du virus.

3. AHSV recombinant atténué vivant selon la revendication 2, dans lequel le nombre de codons d'initiation éliminés par substitution varie entre un et dix.

4. AHSV recombinant atténué vivant selon la revendication 1, dans lequel le virus recombinant a été altéré de sorte que l'expression de NS4 en tant que type sauvage ou protéine complète a été éliminée par suppression d'au moins un codon d'initiation du cadre de lecture ouvert codant pour NS4, sans affecter le fonctionnement de la protéine virale 6 (VP6) du virus.

5. AHSV recombinant atténué vivant selon la revendication 4, dans lequel le nombre de codons d'initiation éliminés par suppression varie entre un et dix.

6. AHSV recombinant atténué vivant selon l'une quelconque des revendications 1 à 5, dans lequel l'ASHV est choisi parmi le groupe constitué du sérotype d'ASHV 1 au sérotype d'AHSV 9, de souches d'AHSV atténuées.

7. AHSV recombinant atténué vivant selon l'une quelconque des revendications 1 à 6, dans lequel le virus recombinant inclut un segment de génome 2 et/ou 6 codant pour les protéines virales (VP2 et 5) déterminant le sérotype d'un autre sérotype ou d'une autre souche d'AHSV.

8. Procédé de production d'un AHSV recombinant atténué vivant non pathogène, le procédé comprenant les étapes consistant à :
- sélectionner une souche particulière d'AHSV ;
- déterminer la séquence génomique de la souche d'AHSV choisie ;
- altérer la séquence génomique de la souche d'AHSV de sorte que l'expression de la protéine non structurelle 4 (NS4) en tant que type sauvage ou de la protéine complète est éliminée ;
- synthétiser des plasmides de transmission contenant la séquence génomique altérée ;
- synthétiser des plasmides d'expression contenant des cadres de lecture ouverts optimisés pour codon des protéines pour fournir un complexe de réplication ;
- synthétiser de l'ARN simple brin (ARNss) à partir desdits plasmides de transcription ;
- transfecter une ligne cellulaire permissive avec lesdits plasmides d'expression ;
- transfecter consécutivement ladite ligne cellulaire permissive transfectée avec l'ARNss ; et
- permettre l'incorporation de l'ARNss à l'intérieur du complexe de réplication dans la ligne cellulaire permissive transfectée pour produire l'AHSV recombinant atténué vivant qui peut se répliquer.

9. Procédé selon la revendication 8, comprenant les étapes supplémentaires consistant à séquencer de nouveau le génome de l'AHSV recombinant produit pour confirmer que l'altération du génome a été effectuée.

10. Procédé selon la revendication 8 ou la revendication 9, comprenant l'étape supplémentaire consistant à remplacer l'ARNss synthétique du segment de génome 2 codant pour la protéine virale (VP2) déterminant le sérotype avec celui d'un autre sérotype d'AHSV.

11. Vaccin comprenant l'AHSV recombinant atténué vivant selon l'une quelconque des revendications 1 à 7.

12. Vaccin selon la revendication 11 incluant un support, un adjuvant ou un véhicule pharmaceutiquement acceptable.

13. Vaccin selon la revendication 12, dans lequel le support pharmaceutiquement acceptable est choisi parmi le groupe constitué des adjuvants à base d'eau et des nanoparticules liquides contenant un composé stimulant l'immunité et une combinaison de ceux-ci.

14. Vaccin polyvalent, le vaccin comprenant un AHSV recombinant atténué vivant d'un sérotype, selon l'une quelconque des revendications 1 à 7, et au moins d'un autre sérotype d'AHSV recombinant atténué vivant, selon l'une quelconque des revendications 1 à 7.

15. Vaccin selon l'une quelconque des revendications 11 à 14, pour une utilisation dans la vaccination des animaux contre l'AHSV.

16. Kit d'immunisation comprenant deux conteneurs, chacun incluant au moins quatre sérotypes différents d'AHSV recombinant selon l'une quelconque des revendications 1 à 7, l'arrangement étant tel que le kit est convenable pour une utilisation dans un procédé d'immunisation d'un animal contre tous les neufs sérotypes d'AHSV.

17. Vaccin selon l'une quelconque des revendications 11 à 14, pour une utilisation dans la vaccination des animaux contre l'AHSV, l'utilisation incluant l'étape consistant à fournir une quantité efficace du vaccin selon l'une quelconque des revendications 11 à 14 à un animal qui en a besoin.
